# EUROPEAN PATENT SPECIFICATION

(11) **EP 3 321 773 B1**
(45) Date of publication and mention of the grant of the patent: **14.12.2022**
(21) Application number: 16821111.8
(22) Date of filing: 25.05.2016
(51) Int. Cl.: G06F 3/01, A63F 13/211, A63F 13/212, A63F 13/213, A63F 13/215, A63F 13/216, A63F 13/218, A63F 13/28, A63F 13/79, G06T 19/00, H04N 21/442, H04N 21/488, A63F 13/35

(54) **INFORMATION PROCESSING DEVICE, DISPLAY DEVICE, INFORMATION PROCESSING METHOD, AND PROGRAM**
INFORMATIONSVERARBEITUNGSVORRICHTUNG, ANZEIGEVORRICHTUNG, INFORMATIONSVERARBEITUNGSVERFAHREN UND PROGRAMM
DISPOSITIF DE TRAITEMENT D'INFORMATIONS, DISPOSITIF D'AFFICHAGE, PROCÉDÉ DE TRAITEMENT D'INFORMATIONS ET PROGRAMME

(30) Priority: 08.07.2015 JP 2015136932
(43) Date of publication of application: 16.05.2018
(73) Proprietor: Sony Group Corporation, Tokyo 108-0075 (JP); Sony Interactive Entertainment Inc., Tokyo 108-0075 (JP)
(72) Inventor: UCHIYAMA, Hiromasa, Tokyo 108-0075 (JP); SUZUKI, Hideyuki, Tokyo 108-0075 (JP); TANUMA, Fumihiko, Tokyo 108-0075 (JP); MIYAZAKI, Yoshio, Tokyo 108-0075 (JP)
(74) Representative: Witte, Weller & Partner Patentanwälte mbB
(86) International application number: PCT/JP2016/065385
(87) International publication number: WO 2017/006640

(56) References cited:
- WO-A1-2014/156389
- WO-A1-2014/156389
- WO-A1-2015/030797
- JP-A- 2000 339 490
- US-A1- 2006 273 984
- US-A1- 2012 194 554
- US-A1- 2013 009 993
- US-A1- 2014 274 564

## Description

### Technical Field

The present disclosure relates to an information processing system.

### Background Art

Developments in technologies for virtual reality (VR) and augmented reality (AR) have enabled users to obtain a sense of deep immersion which is confusing with the real world in a world where information including virtual objects is provided. On the other hand, with a sense of deep immersion, users may become unable to distinguish between a world of virtual reality or augmented reality and the real world. Therefore, document JP 2014-187559A, for example, discloses, in a case where a user commits an error in the border between virtual reality and reality, explicitly presenting again a world of virtual reality that the user has experienced to cause the user to recognize the world of virtual reality. Document US 2012/194554 A1, according to its abstract, provides an apparatus comprising a memory storing instructions. The apparatus includes a control unit for executing the instructions to send signals to display, for a user, a first virtual image superimposed onto an image of real space, the image of real space comprising an image of a potential source of interest for the user. The control unit further executes instructions to send signals to send signals to analyze the image of real space to detect the potential source of interest. The control unit further executes instructions to send signals to notify the user of the potential source of interest. Document US 2014/274564 A1, according to its abstract, provides a systems devices and methods for an exercise gaming platform particularly suited for multiple users. The exercise gaming platform has a variable resistance exercise device operatively coupled to a virtual reality environment rendered by a computer such that user exercise motion on the variable resistance exercise device translates to movement of an avatar representing the user in the virtual reality environment and wherein the virtual reality environment has collision objects capable of a collision with the avatar representing the user in the virtual environment, and wherein the collision of a collision object with the avatar representing the user in the virtual environment causes the resistance level of the user's variable resistance exercise device to change. Also disclosed are methods for providing power to virtual machines and methods for procedurally generating virtual terrain changes in response to changes of resistance on a variable resistance exercise device.

Further relevant background art is provided by US2006/0273984 A1, WO2014/156389 A1 and US2013/0009993 A1.

### Disclosure of Invention

### Technical Problem

However, document JP 2014-187559A above does not cause a user to recognize the border between virtual reality and reality while the user is experiencing a world of virtual reality. Thus, it is not possible to cope with a case in which it is desired to cause a user to immediately recognize the border between virtual reality and reality on the basis of an activity, a comment, or the like of the user who is experiencing a world of virtual reality, for example.

Therefore, the present disclosure proposes an information processing system being improved that, in accordance with a state of a user who is experiencing a world of virtual reality or augmented reality, can cause the user to appropriately recognize the world of virtual reality or augmented reality and the real world.

### Solution to Problem

According to the present disclosure, there is provided an information processing system according to the claim 1.

### Advantageous Effects of Invention

According to the present disclosure as described above, in accordance with a state of a user who is experiencing a world of virtual reality or augmented reality, it is possible to cause the user to appropriately recognize the world of virtual reality or augmented reality and the real world.

### Brief Description of Drawings

[FIG. 1] FIG. 1 is an explanatory diagram showing a schematic configuration of an information processing system according to an embodiment of the present disclosure.
[FIG. 2] FIG. 2 is a functional block diagram showing a functional configuration of the information processing system according to the embodiment.
[FIG. 3] FIG. 3 is a flowchart showing processing in the information processing apparatus in a use case A.
[FIG. 4] FIG. 4 is a flowchart showing setting of measurement timing in a pre-configure case.
[FIG. 5] FIG. 5 is a flowchart showing setting of measurement timing in a configurable case.
[FIG. 6] FIG. 6 is a flowchart showing learning type determination processing in a use case B.
[FIG. 7] FIG. 7 is a flowchart showing feedback processing in the use case B.
[FIG. 8] FIG. 8 is a flowchart showing feedback processing in a use case C and shows a case in which a feedback completion report is made from a user.
[FIG. 9] FIG. 9 is a flowchart showing feedback processing in the use case C and shows a case in which a feedback completion report is made from a third party.
[FIG. 10] FIG. 10 is a flowchart showing feedback processing in a use case D.
[FIG. 11] FIG. 11 is an explanatory diagram showing a display change in accordance with a difference in the number of polygons.
[FIG. 12] FIG. 12 is a correspondence table between the distance from a camera and a model in LOD control of polygon model.
[FIG. 13] FIG. 13 is a functional block diagram of a functional unit that performs LOD control of polygon model.
[FIG. 14] FIG. 14 is a hardware configuration diagram showing a hardware configuration of the information processing apparatus according to the embodiment.

### Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

Note that description will be provided in the following order.
1. Overview
2. Configuration of information processing system
   2.1. State notifying device
   2.2. Server
   2.3. Feedback device
3. Use cases
   3.1. Use case A (in which an active action is received to provide feedback for a user himself/herself)
   3.2. Use case B (in which a user is monitored using a passive action to provide feedback for the user himself/herself)
   3.3. Use case C (in which a third party other than a user performs an active action to provide feedback for the user himself/herself)
   3.4. Use case D (in which a user is monitored using a passive action to provide feedback for a third party)
4. Feedback examples by means of display change
5. Hardware configuration examples

### <1. Overview>

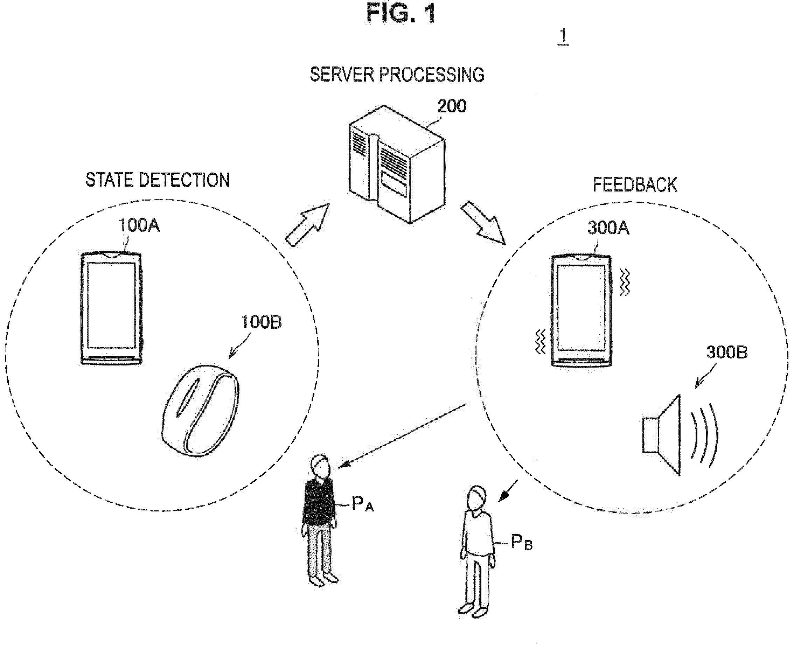

First, with reference to FIG. 1, a schematic configuration of an information processing system 1 according to an embodiment of the present disclosure will be described. Note that FIG. 1 is an explanatory diagram showing a schematic configuration of the information processing system 1 according to the present embodiment.

The information processing system 1 is a system that causes a user who is experiencing a world (hereinafter, referred to as a "virtual world") in which information including virtual objects is provided, such as a world of virtual reality or augmented reality, to differentiate between a virtual world and the real world in accordance with a user state. In the information processing system 1, the state of a user who is experiencing a virtual world is detected, and the necessity for feedback for causing the user to differentiate between the virtual world and the real world is determined in a server in accordance with the user state, and feedback is provided for the user, as shown in FIG. 1.

For example, a user P_{A} who is experiencing a virtual world is considered. At this time, measurement and monitoring of the physical state and psychological state of the user P_{A} are being performed. Monitoring is performed by using a device of a smartphone 100A or a wristband activity monitor 100B held by the user P_{A} or the like, or using an environmentally installed device such as a surveillance camera, for example. Information (hereinafter referred to as "monitoring information" as well) acquired by monitoring is transmitted to a server 200 at predetermined timing. In addition, it is also possible for the user P_{A} himself/herself or a third party P_{B} to actively request the server 200 to provide feedback for the user P_{A}.

The server 200 having received the monitoring information determines a user state of the user P_{A} on the basis of the monitoring information to determine whether feedback for the user P_{A} is necessary or not. When it is determined that feedback for the user P_{A} is necessary, the server 200 produces vibrations or outputs a sound to the device held by the user P_{A} to provide feedback for the user P_{A}. Alternatively, the server 200 may produce vibrations or output a sound to a device such as a smartphone 300A or an audio player 300B held by the third party P_{B} who is able to take an action to the user P_{A} to provide feedback for the user P_{A} from the third party P_{B}, In addition, in a case where a request for feedback has been received from the user P_{A} himself/herself or the third party P_{B}, the server 200 may also determine that feedback for the user P_{A} is necessary, and perform similar processing.

In this manner, the information processing system 1 according to the present embodiment can cause feedback to be provided when it is necessary for a user who is experiencing a virtual world to differentiate between the virtual world and the real world or when the user desires to know. Accordingly, it is possible to prevent the user who is experiencing the virtual world from being excessively immersed in the virtual world to become unable to differentiate between what the user may execute in reality and what the user may execute in the virtual world, which can ensure safety of the user. Hereinafter, a configuration and functions of the information processing system 1 according to the present embodiment will be described in detail.

### <2. Configuration of information processing system>

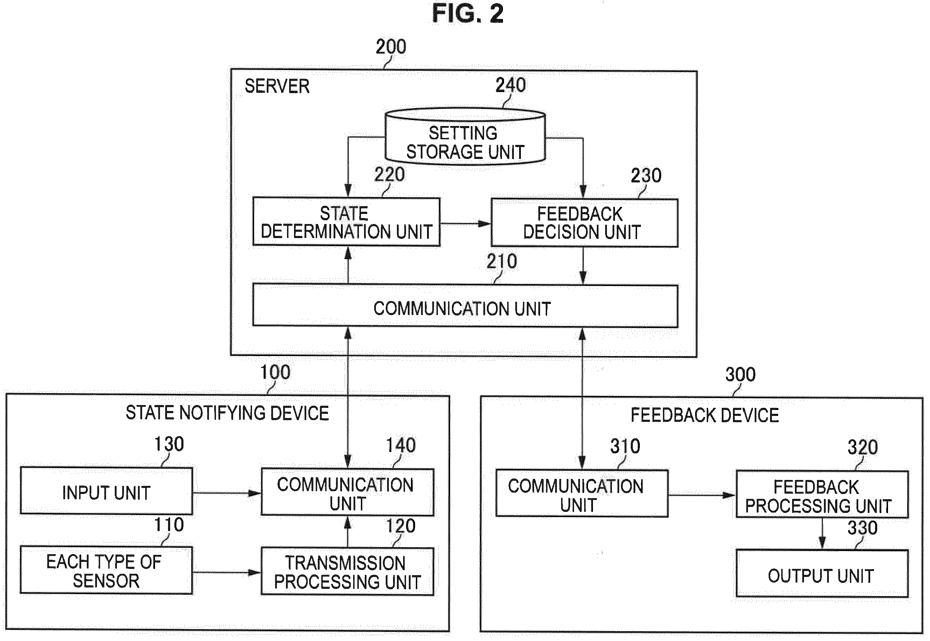

On the basis of FIG. 2, a functional configuration of the information processing system 1 according to the present embodiment will be described. Note that FIG. 2 is a functional block diagram showing a functional configuration of the information processing system 1 according to the present embodiment. The information processing system 1 according to the present embodiment includes a state notifying device 100, the server 200, and a feedback device 300, as shown in FIG. 2. Note that FIG. 2 shows the respective functional units in a manner divided into three devices in order to provide description in association with a flow of processing in the information processing system 1. Consequently, the configuration of the information processing system 1 in the present disclosure is not limited to such an example, but the state notifying device 100 and the feedback device 300 may be the same device, for example.

### [2.1. State notifying device]

### (a) Functions

The state notifying device 100 transmits monitoring information regarding a user who is experiencing a virtual world or feedback request information from the user himself/herself or a third party to the server 200. An action that the state notifying device 100 makes a notification to the server 200 is classified into a "passive action" and an "active action."

### (Passive action)

The "passive action" includes causing gauging or monitoring of information to be used for specifying a user state to be performed in various devices constituting the information processing system 1 and transmitting the information to the server 200. Monitoring is mainly targeted for a biological state or a psychological state. That is, the state notifying device 100 carries out a measurement or monitoring of a physical state or a psychological state of a user who is experiencing a virtual world to acquire information for causing the server 200 to determine execution of feedback for the user.

Monitoring by the state notifying device 100 means gauging a physical state or a psychological state of a user. Monitoring can be classified into continuous monitoring and discrete monitoring. In discrete monitoring, a physical state or a psychological state of a user is measured at certain constant intervals. The frequency and interval of measurement may be set beforehand in the system 1, or may be set in conformity with a specific parameter in the system 1 or the like.

Examples of items to be gauged or monitored include eyes (a point of view, pupil, retina), three semicircular canals, a heart rate, a blood pressure, perspiration, electroencephalogram, positional information, a voice tone, remarks, and the like. Regarding remarks, information such as whether a user who is playing a game asks another player about an intention, a condition check, or the like is acquired, for example.

For efficiently carrying out gauging or monitoring, the state notifying device 100 may carry out not only pre-configuration of setting a monitoring condition beforehand, but also configurable monitoring in which the configuration can be changed adaptively. That is, pre-configured gauging or monitoring is performed on the basis of measuring timing at which a user is gauged or monitored, the measuring timing being set beforehand in the server. The measuring timing may be defined using a value of Duration/Period/Offset, for example.

On the other hand, for configurable gauging or monitoring, the measuring timing at which the user is gauged or monitored may be set dynamically on the server side in accordance with a function status of the system 1. Examples of the function status of the system 1 include a status of an item under gauging/monitoring, a network status, communication quality, a congestion status, a device status such as a battery status, and the like. The state notifying device 100 can perform configurable gauging or monitoring by reflecting such a function status of the system 1 to the value of Duration/Period/Offset that defines the measuring timing, for example.

### (Active action)

Examples of the "active action" include an action in which a user himself/herself who is experiencing a virtual world or a third party makes a request for feedback for the user for differentiating between the virtual world and the real world.

Examples of a situation in which the user himself/herself requests feedback include a case in which the user becomes unable to distinguish whether the world he/she is experiencing is the real world or the virtual world, and desires to know which world he/she is experiencing, and the like. A feedback request by the user himself/herself may be performed by pressing down a button for transmitting a feedback request to the server 200, for example. This button may be a physical button existing in the real world, or may be a button existing in the virtual world as a virtual object.

In addition, a feedback request may be transmitted to the server 200 when the user utters a predetermined keyword, for example. At this time, the state notifying device 100 voice recognizes the user's utterance, and detects the keyword. For example, the keyword may be set beforehand by the user himself/herself, or may be set beforehand in the system 1. Specifically, a keyword such as a "current situation recognition request" may bet set. Further, a feedback request may be transmitted to the server 200 when the user makes a predetermined gesture.

On the other hand, examples of a situation in which a third party makes a feedback request include a case in which it is assumed that the user himself/herself is unable to differentiate between the virtual world and the real world, or the like, such as when the user is hardly conscious without the user himself/herself knowing it. That is, a third party is able to request the server 200 to provide feedback for the user in order to help the user who is unable to differentiate between the virtual world and the real world. Here, the third party is a person who is able to execute an action for the user in the real world or the virtual world. For example, in a case where the user is playing a game provided in the virtual world, the third party who requests feedback for the user may be a person present around the user in the real world, or may be a person virtually interacting with the user who is a player in the virtual world.

For example, the feedback request by the third party may be made by pressing down a button or inputting a command using a device such as a game controller, making a predetermined gesture, or transmitting feedback request information from a communication device such as a smartphone held by the third party.

### (b) Device configuration example

Such a state notifying device 100 may include each type of sensor 110, a transmission processing unit 120, an input unit 130, and a communication unit 140, as shown in FIG. 2, for example. Specifically, the state notifying device 100 may be a smartphone held by the user, a wearable terminal such as a wristband activity monitor or an eyewear terminal, or the like, for example. Alternatively, the state notifying device 100 may be an environmentally installed device such as surveillance camera.

Each type of sensor 110 is a functional unit that performs gauging or monitoring of information to be used for specifying a user state. Each type of sensor 110 is a sensor for acquiring biological information, such as an acceleration sensor, an angular velocity sensor, a barometric sensor, or a heart-beat sensor, a GPS, a microphone, an imaging sensor, or the like, for example. Each type of sensor 110 acquires a user state at predetermined measuring timing, and outputs the user state to the transmission processing unit 120.

The transmission processing unit 120 performs processing of transmitting the information for specifying a user state acquired by each type of sensor 110 to the server 200 via the communication unit 140. The transmission processing unit 120 transmits the information acquired by each type of sensor 110 to the server 200 at predetermined timing.

The input unit 130 is a functional unit for the user to perform an operation input. In the present embodiment, the input unit 130 is utilized particularly when the user makes a feedback request. The input unit 130 includes an operation input unit such as a button, a keyboard, a lever, a switch, or a touch sensor, and an information acquisition unit such as a microphone that acquires voice, an imaging sensor that acquires an image, and the like, for example. Information acquired by the input unit 130 corresponds to a user's feedback request, and is transmitted to the server 200 via the communication unit 140. The communication unit 140 is a functional unit for enabling information transmission/reception to/from the server 200.

Note that, in the state notifying device 100 shown in FIG. 2, each type of sensor 110 and the transmission processing unit 120 for detecting a passive action and the input unit 130 for detecting an active action are provided in the same device, whilst the present disclosure is not limited to such an example. For example, each type of sensor 110 and the transmission processing unit 120 may be provided in a device different from that of the input unit 130, so that information is transmitted from a plurality of state notifying devices 100 to the server 200.

### [2.2. Server]

### (a) Functions

### (Feedback necessity determination)

The server 200 determines whether or not to provide feedback for a user on the basis of information received from the state notifying device 100. For example, the determination of necessity for feedback for the user may be an instantaneous type determination for making a determination according to whether a measured value has exceeded a predetermined threshold value or not, or may be a learning type determination for determining the necessity for feedback by learning using various types of data acquired in the state notifying device 100.

The instantaneous type determination is applicable to both an active action and a passive action transmitted from the state notifying device 100. Note that, in a case of determining a passive action, an instantaneous determination is not necessarily appropriate in some cases. For example, in a case of gauging a tension state of a user when the user is playing a game provided in a virtual world, the server 200 needs to appropriately determine whether the user the user is in a tension state temporarily or not in accordance with contents of the game. Since such a determination is difficult in the instantaneous type determination, it is better to utilize the learning type determination for a statistic determination of a tension state.

On the other hand, the learning type determination learns a measurement result by each type of sensor 110 acquired by accumulated passive actions, and sets a determination criterion to decide the necessity for feedback from a learning result. Then, the server 200 determines whether the current user state is a state in need of feedback or not on the basis of the determination criterion set from the learning result. Note that details of the learning type determination will be described later.

### (Feedback means)

When it is decided in the server 200 to provide feedback for the user, how to provide feedback is decided. Patterns of feedback are broadly divided into a case of directly providing for the user himself/herself and a case of making a notification to a third party and providing feedback for the user from the third party having received the notification. There is one or more means for receiving feedback or a notification from the server 200, and a plurality of means may be used in combination.

First, in a case of directly providing feedback for the user himself/herself, it can be done via a device held or worn by the user or a virtual world that the user is experiencing.

For example, feedback may be provided for the user with a device such as a smartphone or a wearable terminal. Feedback may be provided by turning off the device, outputting a sound for causing the real world and the virtual world to be differentiated from a speaker, or vibrating the device, for example. In addition, feedback may be provided by presenting information that stimulates a perception, such as pain, itch, intraocular pressure, or smell to the user, or utilizing a kind of medicine that inhibits a user's motion, such as an anesthetic or a hypnotic drug. Further, feedback may be provided by hiding virtual objects, or changing a display of the virtual world to a see-through image of a camera to present the real world alone to the user.

How to provide feedback may be decided in the server 200 in accordance with functions included in a device that provides feedback, and the server 200 only transmits a feedback execution instruction, and a decision may be made on the device side having received the instruction.

In addition, in a case of providing feedback via a virtual world that the user is experiencing, such as when the user is playing a game provided in the virtual world, feedback may be provided visually, acoustically, or the like. For example, text or a specific object indicating that it is the virtual world may be displayed as a virtual object, or a notification that it is the virtual world may be made by voice. In addition, in a case where the number of polygons of virtual objects constituting the virtual world is large, and it is difficult to differentiate them from things in the real world, the number of polygons of the virtual objects may be reduced to change a display, such that the real world and the virtual world can be differentiated. Alternatively, it is also possible to cause the user to differentiate between the real world and the virtual world by displaying the virtual objects in the virtual world with frames. In addition, a story of a content provided as the virtual world may be changed to cause the user to differentiate between the real world and the virtual world.

On the other hand, in a case of providing feedback for the user from a third party other than the user, an instruction to provide feedback for the user is transmitted to a device held or worn by the third party, and then the third party makes contact with the user to provide feedback for the user. Here, as the third party, a relative such as user's parents or family, a friend, people present around the user in the real world, a corporation such as a game operating company or an insurance company, a public authority such as the police or emergency, a player virtually interacting with the user in the virtual world, or the like, for example, is assumed.

For example, in a case of issuing a feedback instruction to a relative such as user's parents or family, or a friend, the server 200 transmits information concerning feedback to a device that the user has registered in advance. Examples of information concerning feedback include warning information for the user, user's positional information, urgency of feedback, and the like. Alternatively, in a case where a device worn by the user himself/herself is provided with a notification unit, such as a display, that is visible to surrounding people, text, an image, or the like that requests surrounding people to provide feedback may be displayed on the notification unit. When a relative such as user's parents or family, or a friend becomes aware that the device worn by the user is making a notification that requests feedback, feedback can be provided for the user.

In addition, in a case of issuing a feedback instruction to a person present around the user in the real world, for example, the server 200 specifies a person present around the user on the basis of user's positional information, and transmits information concerning feedback to a device held by the specified person. Examples of information concerning feedback include warning information for the user, user's positional information, urgency of feedback, user profile information for identifying the user, such as the user's face and sex, and the like. In addition, also in this case, in a case where the device worn by the user himself/herself is provided with a notification unit, such as a display, that is visible to surrounding people, text, an image, or the like that requests surrounding people to provide feedback may be displayed on the notification unit.

Further, in a case of issuing a feedback instruction to a corporation such as a game operating company or an insurance company, for example, the server 200 transmits information concerning feedback via servers of these corporations. Examples of information concerning feedback include warning information for the user, user's positional information, urgency of feedback, user profile information for identifying the user, such as the user's face and sex, and the like, for example. In addition, the server 200 may request providing feedback for the user via the virtual world, and may cause text, a sound, or an object for differentiating between the real world and the virtual world to be displayed in the virtual world, or may change a setting or story of a content provided as the virtual world.

In addition, in a case of issuing a feedback instruction to a public authority such as the police or emergency, for example, the server 200 also transmits information concerning feedback via servers of these public authorities. Examples of information concerning feedback include warning information for the user, user's positional information, urgency of feedback, user profile information for identifying the user, such as the user's face and sex, and the like, for example.

Further, in a case of issuing a feedback instruction to a player who is virtually in contact with the user in the virtual world, for example, the server 200 instructs to provide feedback for the user via the virtual world, for example. The player having received this makes contact with the user in the virtual world, and provides feedback for the user by text, a sound, a display change, or the like. In addition, the player may provide feedback for the user via the real world. For example, feedback is provided for the user by causing sound representing warning contents to be output from a user's wear, or changing a display of virtual objects in the virtual world that the user is experiencing. At this time, for example, processing of reducing the number of polygons of the virtual objects may be performed or the virtual objects may be displayed with frames, so that a difference from corresponding objects in the real world can be clarified.

### (b) Device configuration example

Such a server 200 includes a communication unit 210, a state determination unit 220, a feedback decision unit 230, and a setting storage unit 240, as shown in FIG. 2, for example.

The communication unit 210 transmits/receives information to/from the state notifying device 100 and the feedback device 300 which will be described later. The communication unit 210 outputs information received from the state notifying device 100 to the state determination unit 220, and transmits information input from the feedback decision unit 230 to the feedback device 300.

The state determination unit 220 determines a user state for determining whether feedback for the user is necessary or not on the basis of information received from the state notifying device 100. For example, the state determination unit 220 performs a learning type determination of determining a user state by learning on the basis of a measurement result of each type of sensor 110 that the state notifying device 100 has acquired as a passive action, or an instantaneous type determination of determining a user state according to whether a predetermined measured value has exceeded a threshold value or not. In addition, the state determination unit 220 may instantaneously determine the necessity for feedback for the user according to whether feedback request information from the user himself/herself or a third party has been received from the state notifying device 100 or not.

A determining method of the state determination unit 220 is set in accordance with information received from the state notifying device 100 depending on the configuration of the information processing system 1. Consequently, the state determination unit 220 does not necessarily need to include both functions of the instantaneous type determination and the learning type determination, but it may be configured to be capable of determining the necessity for feedback for the user by at least one of the determining methods. When it is determined by the state determination unit 220 that feedback for the user is necessary, the feedback decision unit 230 is notified to that effect.

On the basis of a user state of a user who is experiencing a world in which information at least partially including a virtual object is provided, the feedback decision unit 230 decides feedback for the user. When it is decided to provide feedback for the user, the feedback decision unit 230 decides how to provide feedback. Patterns of feedback are broadly divided into a case of directly providing for the user himself/herself and a case of making a notification to a third party and providing feedback for the user from the third party having received the notification. There is one or more means for receiving feedback or a notification from the server 200, and a plurality of means may be used in combination. How to provide feedback may be decided in the feedback decision unit 230 in accordance with functions included in a device that provides feedback. Alternatively, the feedback decision unit 230 may transmit a feedback execution instruction alone, and a decision may be made on the side of a device (the feedback device 300 which will be described later) having received the instruction.

The feedback decision unit 230 transmits feedback information necessary for providing feedback to a device (the feedback device 300) of the user or a third party or the like via the communication unit 210.

The setting storage unit 240 holds threshold value information utilized when determining the necessity for feedback for the user performed in the server 200 and information concerning feedback to be transmitted to the user himself/herself or a third party when providing feedback for the user. The state determination unit 220 and the feedback decision unit 230 execute their processing referring to the setting storage unit 240.

### [2.3. Feedback device]

### (a) Functions

The feedback device 300 directly provides feedback for a user on the basis of information concerning feedback received from the server 200, or causes a third party to activate to provide feedback for the user. In a case where feedback is directly provided for the user himself/herself, the feedback device 300 may be the same device as the state notifying device 100. In addition, in a case of instructing the third party to provide feedback for the user, the feedback device 300 is a device held or worn by the third party, or a server of a corporation, for example.

### (b) Device configuration example

The feedback device 300 includes a communication unit 310, a feedback processing unit 320, and an output unit 330, as shown in FIG. 2, for example.

The communication unit 310 transmits/receives information to/from the server 200. The communication unit 310 outputs information concerning feedback received from the server 200 to the feedback processing unit 320.

The feedback processing unit 320 performs information processing for providing feedback for the user or a third party from the feedback device 300 having received the information concerning feedback. Feedback may be provided in the real world, or may be provided in the virtual world.

For example, in a case of directly providing feedback for a user who is experiencing a virtual world, the feedback processing unit 320 performs processing of turning off a device, outputting a sound from a speaker, or vibrating the device, in the real world. In addition, the feedback processing unit 320 may perform processing of presenting information that stimulates a user's perception, or giving the user a kind of medicine that inhibits the user's motion, such as an anesthetic or a hypnotic drug. Further, the feedback processing unit 320 may perform processing of hiding virtual objects, or changing a display of the virtual world to a see-through image of a camera to present the real world alone to the user. Upon receiving a result of processing of the feedback processing unit 320, feedback for the user is output from the output unit 330.

In addition, in a case of providing feedback for the user via the virtual world, the feedback processing unit 320 performs processing of making contact with the user in the virtual world to provide feedback for the user by text, a sound, a display change, or the like. At this time, the feedback processing unit 320 may perform processing of reducing the number of polygons of the virtual objects or may display the virtual objects with frames, for example, so that a difference from corresponding objects in the real world can be clarified.

On the other hand, in a case of instructing a third party to provide feedback for the user, the feedback processing unit 320 performs processing of notifying the third party of information for specifying the user and urgency on the basis of information concerning feedback received from the server 200. The information concerning feedback includes warning information for the user, user's positional information, and urgency of feedback. In addition, in a case where the third party is a person who does not know the user, the information concerning feedback includes user profile information for identifying the user, such as the user's face and sex, and the like.

Note that in a case of causing the third party to provide feedback for the user, and in a case where a device worn by the user himself/herself is provided with a notification unit that is visible to surrounding people, text, an image, or the like that requests surrounding people to provide feedback may be displayed on the notification unit. In this case, an alarm, vibrations, or the like that prompts attention to the surroundings may be presented to the third party in order to make surrounding people quickly notice that a notification requesting feedback is being made from the device worn by the user.

The output unit 330 outputs information on the basis of a result of processing performed by the feedback processing unit 320. The output unit 330 is a display, a speaker, a lamp, or the like, for example. In a case where feedback is directly provided for the user himself/herself, output information from the output unit 330 becomes feedback for the user. In addition, in a case where feedback for the user is instructed to a third party, the third party specifies the user on the basis of information notified from the output unit 330, and provides feedback.

### <3. Use cases>

Hereinafter, configuration examples of the information processing system 1 according to the present embodiment will be shown. Representative configuration examples are shown below, and the present disclosure is not limited to the configuration examples, but functions that can be implemented by the state notifying device 100, the server 200, and the feedback device 300, respectively, can be combined to create an information processing system. Accordingly, in the information processing system 1 according to the present embodiment, it is possible to cause the real world and the virtual world to be differentiated when the necessity actually arises or when a user desires to know while achieving balance between a sense of immersion immersed in the virtual world and a sense of reality, and it is possible to provide a service for protecting the user in a state of being excessively immersed in the virtual world from danger.

### [3.1. Use case A (in which an active action is received to provide feedback for a user himself/herself)]

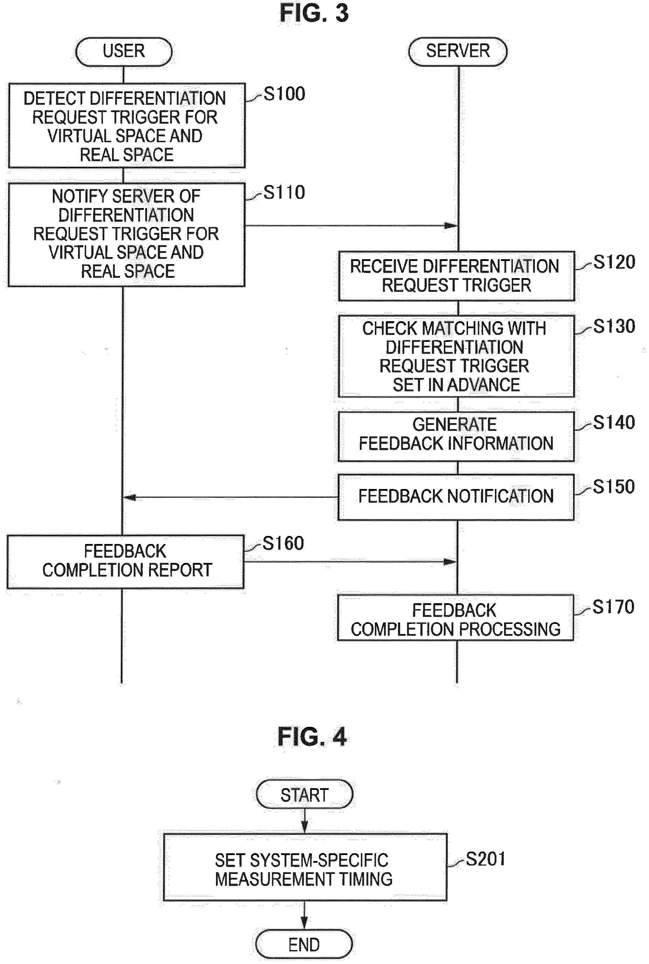

First, on the basis of FIG. 3, a use case where, in a case where a user himself/herself actively makes a feedback request (hereinafter, referred to as a "differentiation request trigger" in the present use case) for differentiating between a virtual world and the real world, the server 200 instantaneously determines the differentiation request trigger, and provides feedback such as a warning for the user himself/herself will be described. Note that FIG. 3 is a flowchart showing processing in the information processing system 1 in the present case. In FIG. 3, processing of a "user" indicates processing in the state notifying device 100 and the feedback device 300.

In the present use case, a situation in which the user becomes unable to differentiate between the virtual world and the real world while being immersed in a game, a network service, or the like of a virtual reality type, an augmented reality type, or the like will be assumed.

First, the input unit 130 of the state notifying device 100 is in a state of always waiting for an input of a differentiation request trigger from the user. As the differentiation request trigger from the user, the following interactions, for example, are assumed.
▪ Operation input via a physical interface:
   pressing down of a physical button in the real world allocated to a physical controller or a button which is a virtual object such as a user interface in the virtual world
▪ Detection of a keyword uttered by the user by voice input/voice recognition:
   detection of a keyword registered beforehand by the user or a keyword set beforehand on the system
▪ Acquisition of a user's gesture:
   detection of a gesture pattern registered beforehand by the user or a gesture pattern set beforehand on the system.

When an input of information is received (S100), the input unit 130 of the state notifying device 100 transmits the information as input data to the server 200 via the communication unit 140 (S110). The server 200 having received the input data (S120) performs an instantaneous type determination whether the input data matches differentiation request trigger data set beforehand or not by the state determination unit 220 (S130).

Note that differentiation request trigger data may have a plurality of patterns, and various levels of feedback information may be allocated to each of the patterns. In this case, in accordance with differentiation request trigger data matched with the input data, contents of feedback for the user may be varied. In addition, an example of determining execution of feedback only on the basis of an active action is described in the present use case, whilst the present disclosure is not limited to such an example, but an active action and a passive action may be combined to make a determination. For example, when an active action is performed, heart rate information gauged as a passive action is also acquired. Then, a feedback method may be changed between a case in which the heart rate is higher than or equal to a predetermined value and a case in which the heart rate is less than the predetermined value when an active action is performed.

Returning to the description of FIG. 3, in a case where it is determined that a request for differentiating between the virtual world and the real world has been made from the user, the server 200 generates feedback information indicating contents of feedback for the user by the feedback decision unit 230 (S140), and provides feedback (S150). For feedback for the user, contents as described below are assumed, for example.
▪ Additional information type feedback:
   giving additional information for the user himself/herself to differentiate between the virtual world and the real world, such as adding frames to virtual objects in the virtual world
▪ Information reduced type feedback:
   deleting a virtual object display in the virtual world, and switching to the screen of the real world alone
▪ Forced termination type feedback:
   forcibly shutting down a content (game/application) of the virtual world.

In this manner, the feedback method can be set in various manners, but stronger feedback is provided for the user in the descending order of the additional information type feedback, the information reduced type feedback, and the forced termination type feedback. Which feedback method is to be used may be decided in accordance with urgency of feedback, for example. By providing stronger feedback as urgency is higher, it is possible to reliably ensure safety of the user immersed in the virtual world. On the other hand, in a case where urgency of feedback is not high, the additional information type feedback, for example, may be provided to allow the user to naturally recognize feedback while enjoying the virtual world so as not to interfere with the state of the user enjoying the virtual world. Urgency of feedback may be determined on the basis of the heart rate, blood pressure, or the like of the user measured by each type of sensor 110 of the state notifying device 100, for example.

After feedback is provided from the server 200 to the feedback device 300, a feedback completion notification indicating that the user has recognized feedback may be transmitted to the server 200 (S160), as shown in FIG. 3. The feedback completion notification may be input from the input unit 130 of the state notifying device 100 similarly to the differentiation request trigger. The feedback completion notification may include a completion notification processing request such as resetting a feedback result, for example. Accordingly, in a case where the user becomes able to distinguish between the virtual world and the real world through the current processing, it is possible to allow the user to return again to the virtual world to enjoy the augmented reality/virtual reality space. The server 200 performs a feedback completion operation in accordance with contents of the request (S170).

### [3.2. Use case B (in which a user is monitored using a passive action to provide feedback for the user himself/herself)]

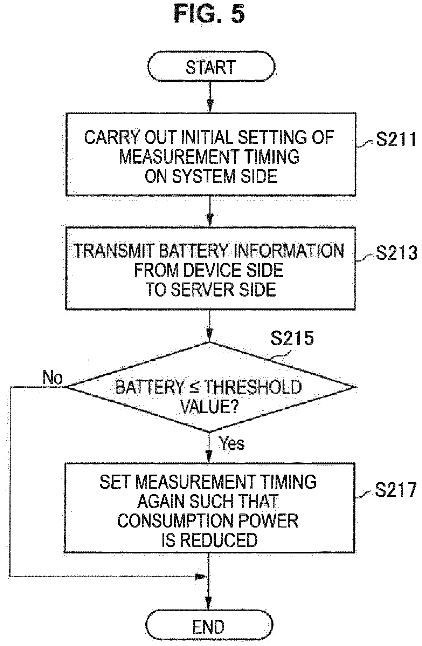

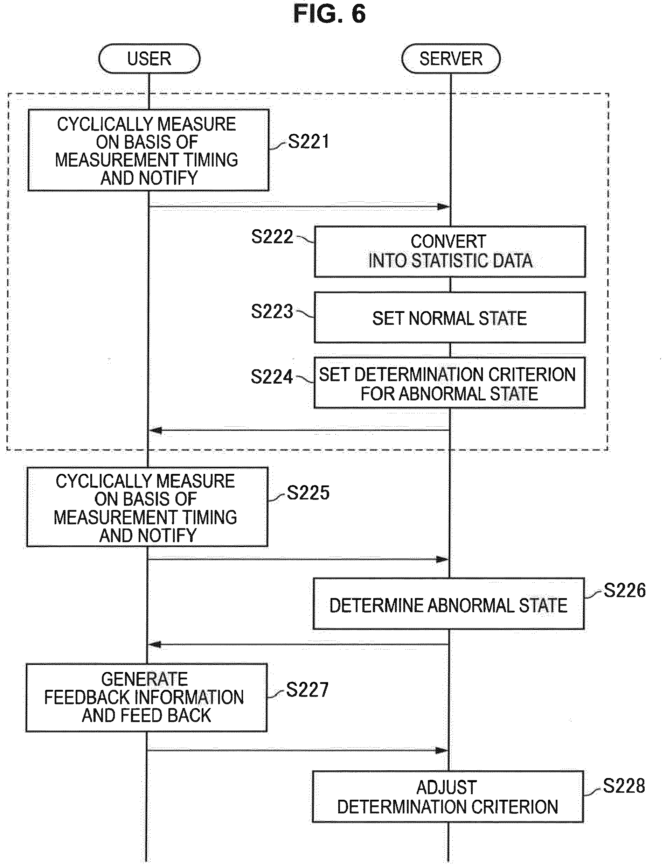

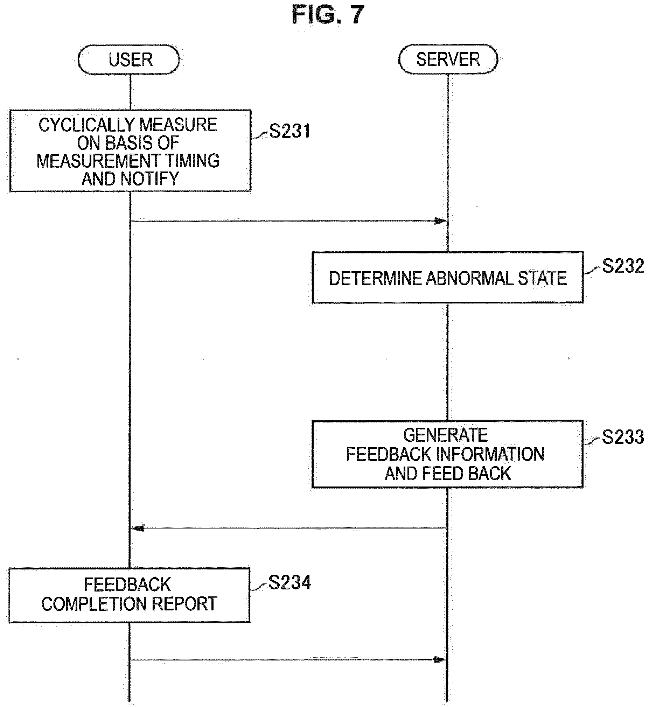

Next, on the basis of FIG. 4 to FIG. 7, a use case in which a user state is monitored cyclically to provide feedback for a user himself/herself on the basis of the learning type determination by the server 200 will be described. Note that FIG. 4 is a flowchart showing setting of measurement timing in a pre-configure case. FIG. 5 is a flowchart showing setting of measurement timing in a configurable case. FIG. 6 is a flowchart showing learning type determination processing in the present use case. FIG. 7 is a flowchart showing feedback processing in the present use case. In FIG. 6 and FIG. 7, processing of the "user" indicates processing in the state notifying device 100 and the feedback device 300.

In the present use case, a situation in which a user is in a dangerous state, such as a state in which the user is unable to differentiate between a virtual world and the real world, for example, while playing a game of a type immersed in the virtual world will be assumed. Note that the present use case may be other than a game, and a use case of Internet of Things (IoT), M2M, or the like, for example, may also be assumed.

First, in order to measure a user state, setting of measuring timing of the user state is performed in the information processing system 1. In the present use case, this measuring timing will be referred to as "measurement timing." In general, measurement timing has the following tradeoffs.
▪ As a shorter interval is set, the user state is measured more accurately; however, a measurement time in the device increases, and CPU/memory consumption and power consumption increase.
▪ As a longer interval is set, the measurement time decreases, and a burden on the device is reduced; however, user state measuring accuracy is lowered.

Thus, it is necessary to set the measurement timing appropriately. Therefore, the measurement timing may be pre-configured, or may be set to be configurable. For example, in the pre-configure case, a parameter is set in advance in the system (S201), as shown in FIG. 4. Examples of the parameter include in what cycle and for what period a measurement is to be performed (Period/Duration/Offset). By performing system-specific setting, signaling overhead can be reduced.

On the other hand, in the configurable case, the measurement timing in the device (the state notifying device 100) is set in the system dynamically or quasistatically. Examples of a parameter for specifically deciding the measurement timing include a battery status of the device. As shown in FIG. 5, for example, after initial setting is carried out in the system 1 (S211), battery information representing a battery capacity is provided from the device for the server 200 (S213). The server 200 determines whether the battery capacity of the device is less than or equal to a threshold value or not on the basis of the received battery information (S215), and when it is recognized that the battery capacity is less than or equal to the threshold value and is small, the cycle and period of the measurement timing are reduced (S217). Accordingly, it is possible to suppress battery consumption of the device. Besides, in the configurable case, it is possible to adaptively change the measurement timing in accordance with a communication status or a measuring target (whether to measure the heart rate or to measure the blood pressure, or the like).

After setting the measurement timing, the device (the state notifying device 100) makes a measurement at settled timing. Examples of a measuring target herein include the heart rate, blood pressure, and the like. The device notifies the server 200 of a measured result at any time.

The server 200 having received data from the device determines a user state by the learning type determination in the present use case. As shown in FIG. 6, the server 200 having received data from the device (S221) accumulates the received data, and converts the data into statistic information by the state determination unit 220 (S222). For example, the state determination unit 220 converts the received data into statistic information such as an average value or a variance value. Then, the state determination unit 220 sets a "normal state" of the user on the basis of the statistic information (S223). For example, the "normal state" may be set as "being less than or equal to ±x from the average value for a certain prescribed time" or the like.

Then, the state determination unit 220 sets a determination criterion for an "abnormal state" (S224). The determination criterion for the "abnormal state" may be set beforehand, or may be set dynamically. For example, the "abnormal state" may be set as a state in which "there is a certain change or more for a certain period" or the like.

Thereafter, when the server 200 receives data from the device (S225) and determines the user state on the basis of the above-described determination criterion, it is assumed that it has been determined that the user is in the "abnormal state" (S226). At this time, the server 200 provides feedback for the device. Here, it is temporarily assumed that the user state is not the "abnormal state" actually although it is determined as the "abnormal state" in step S226. In this case, the device can provide feedback for the server 200 (S227). The server 200 having received feedback about an error in the user state determination from the device can adaptively change the setting of the determination criterion in accordance with this feedback information (S228). In this manner, the server 200 recognizes the "normal state" of the user using statistic data conversion, and learns the determination criterion on the basis of feedback information about a user state determination from the user.

In such an information processing system 1, as shown in FIG. 7, the server 200 receives data from the device (S231), and determines the user state on the basis of the above-described determination criterion. As a result, when it is determined that the user is in the "abnormal state" (S232), the server 200 provides feedback for the user (S233). As feedback for the user, for example, producing a sound or vibrations through the device that the user is using is conceivable. After the feedback, the user carries out a feedback completion notification to the server 200 (S234).

### [3.3. Use case C (in which a third party other than a user performs an active action to provide feedback for the user himself/herself)]

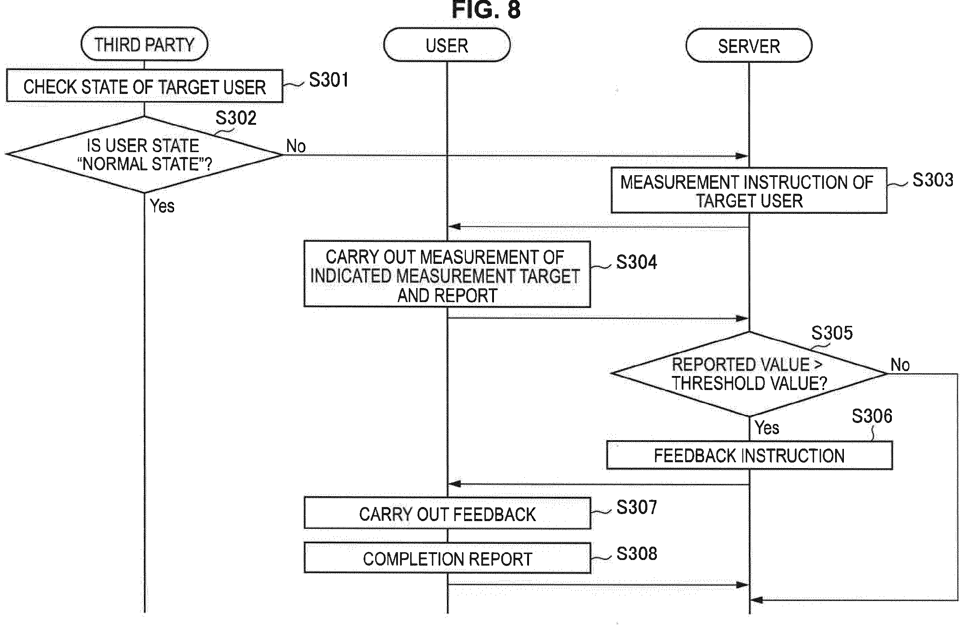

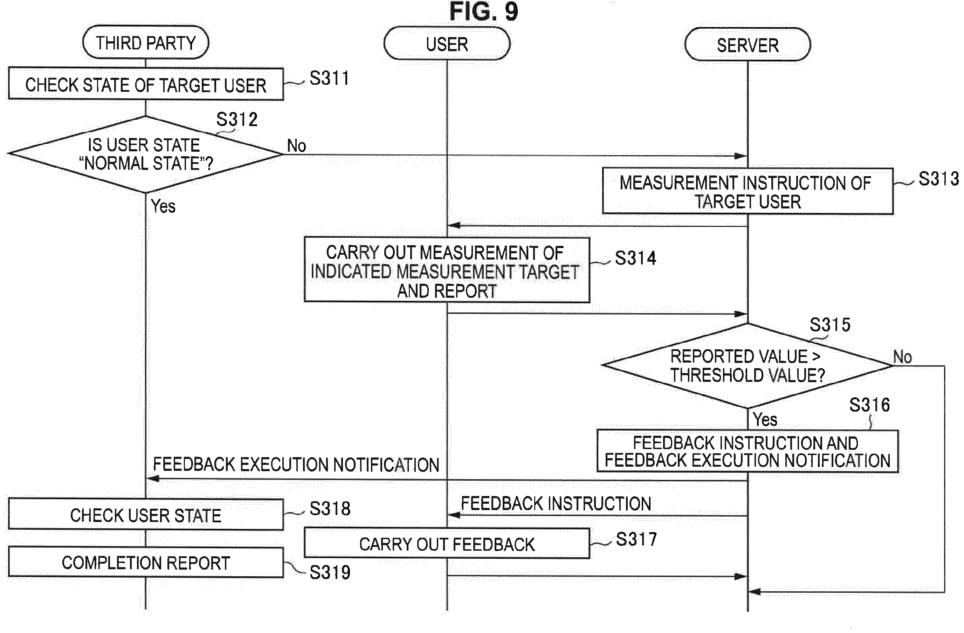

Next, on the basis of FIG. 8 and FIG. 9, a use case in which a third party other than a user who is experiencing a virtual world requests a measurement of the user, and a user state is determined by the server 200 to provide feedback for the user himself/herself will be described. Note that FIG. 8 is a flowchart showing feedback processing in the present use case, and shows a case in which a feedback completion report is made from a user. FIG. 9 is a flowchart showing feedback processing in the present use case, and shows a case in which a feedback completion report is made from a third party. In FIG. 8 and FIG. 9, processing of "user" and "third party" indicate processing in the state notifying device 100 and the feedback device 300.

In the present use case, a situation in which, while a user is playing a game of a type immersed in a virtual world, a third party senses an abnormality of an activity of the user and makes a report to the server 200 will be assumed. The server 200 having received the report from the third party starts monitoring the target user, and if there is an abnormality, provides feedback for the user himself/herself or the third party.

### (Case in which a feedback completion report is made from a user)

First, as shown in FIG. 8, in the present use case, when the third party feels an abnormality of an activity of the user who is experiencing a virtual world, and determines that it is necessary to check the user state, the third party checks the user state visually or the like (S301). A determination herein whether the user state is the "normal state" or not may be made on the basis of the following determination criterion for the "abnormal state", for example.
▪ When another player playing in the same game talks to the user through a game character, there is no reply for a certain time, or there is definitely a strange feeling in conversation
▪ An activity of a player in the real world is definitely outrageous.

Then, in a case where it is determined that the user state is not the "normal state", the third party notifies the server 200 of a measurement request (No in S302).

The server 200 having received the request from the third party carries out a measurement of the user (S303). As a measuring target in the present use case, the blood pressure, heart rate, or the like, for example, is conceivable. The device of the user having received an instruction from the server 200 carries out a measurement, and reports a measured value to the server 200 (S304).

Then, the server 200 determines whether the value reported from the device of the user has exceeded a threshold value (S305), and in a case where the reported value has exceeded the threshold value, provides feedback for the user himself/herself (S306, S307). Examples of feedback for the user include a method such as deleting a content of the virtual world being provided for the user, or changing video presented to the user from a content screen on which virtual objects are presented to a see-through screen to switch to the real world. In addition, for example, a method of activating a function of forcibly stopping the provision of the virtual world from the outside, such as a safety button, to forcibly shut down a game in the virtual world, or the like is also conceivable. After carrying out feedback, the device of the user sends back a feedback completion report to the server 200 (S308).

### (Case in which a feedback completion report is made from a third party)

Next, on the basis of FIG. 9, a case in which a feedback completion report is made from a third party will be described. Processing in steps S311 to S315 in FIG. 9 is the same as steps S301 to S305 in FIG. 8. As shown in FIG. 9, when the third party feels an abnormality of an activity of the user who is experiencing the virtual world, and determines that it is necessary to check the user state, the third party checks the user state (S311). Then, in a case where it is determined that the user state is not the "normal state", the third party notifies the server 200 of a measurement request (No in S312).

The server 200 having received the request from the third party carries out a measurement of the user (S313). The device of the user having received an instruction from the server 200 carries out a measurement, and reports a measured value to the server 200 (S314). Then, the server 200 determines whether the value reported from the device of the user has exceeded a threshold value (S315).

In a case where the reported value has exceeded the threshold value in step S315, the server 200 provides feedback for the user himself/herself (S317), and also notifies the third party that feedback has been provided for the user (S318). The third party having received the notification that feedback has been carried out checks the user state again, and sends back a feedback completion report to the server 200 (S319).

### [3.4. Use case D (in which a user is monitored using a passive action to provide feedback for a third party)]

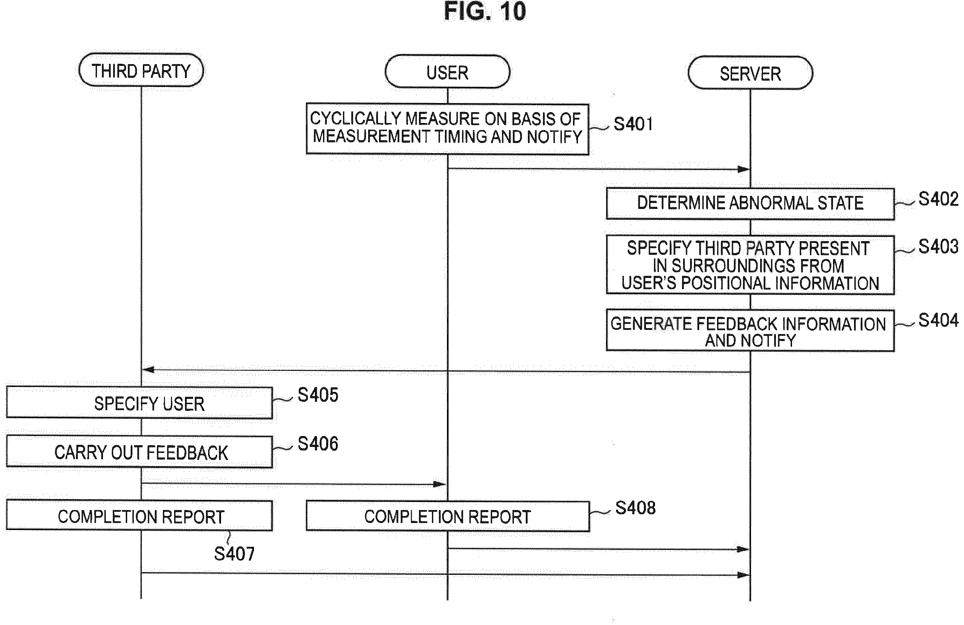

Next, on the basis of FIG. 10, a use case in which a state of a user who is experiencing a virtual world is cyclically monitored, and in a case where an abnormality in the user state is found as a result of the learning type determination by the server 200, feedback is provided for a third party will be described. Note that FIG. 10 is a flowchart showing feedback processing in the present use case. Note that, in FIG. 10, processing of "user" and "third party" indicates processing in the state notifying device 100 and the feedback device 300, respectively.

### (Overview)

In the present use case, a situation in which the user is caught in a situation in which the user is hardly conscious while playing a game of a type immersed in a virtual world, so that the user is unable to make a determination on his/her own will be assumed. In such a situation, since improvement will not be expected even if feedback is provided for the user himself/herself, a warning shall be issued to the user through a third party. Note that, in the present use case, since a measurement performed for performing the user state and the learning type determination processing in the server 200 are similar to those in the use case B, detailed description thereof will be omitted, and processing of providing feedback for a third party will be mainly described.

As sown in FIG. 10, it is assumed that a user state is cyclically measured, and measured data is transmitted to the server (S401). Here, it is assumed that the server 200 makes a learning type determination about the received data, and determines that the user state is the "abnormal state" (S402). At this time, the user specifies a user who is able to provide feedback for the user (S403). Examples of the third party herein include people present around the user in the real world. Specification of the third party is performed on the basis of user's positional information.

When the third party present around the user is specified, the server 200 generates feedback information that commissions to provide feedback for the user, and transmits the feedback information to the device of the third party (S404). Commissioning information that commissions a warning to the user as a feedback target and detailed information regarding the user are included in the feedback information to the third party, for example. Here, warning information for the user, user's positional information, urgency, user profile information, and the like, for example, are included in the detailed information regarding the user. The warning information refers to contents of feedback to be provided for the user, and has contents such as restoring the user to consciousness, for example. The user's positional information is used to specify a detailed position of the user. The urgency indicates what situation the user is in at present and to what degree of urgency feedback needs to be provided. The user profile information is information regarding a profile concerning the user and includes information such as user's sex and face information, a feedback history in the past, and a chronic illness.

The third party finds the user as a target on the basis of these pieces of detailed information (S405), and provides feedback (S406). After completion of the feedback, a feedback completion notification is made from the user or the third party to the server 200 (S407, S408).

### <4. Feedback examples by means of display change>

In the information processing system 1 according to the present embodiment, when providing feedback for a user himself/herself, it is possible to cause the user to differentiate between a virtual world and the real world without completely stopping the provision of the virtual world that the user is experiencing. That is, the user is caused to differentiate between the virtual world and the real world in a manner not to interfere with the user's sense of immersion in the virtual world.

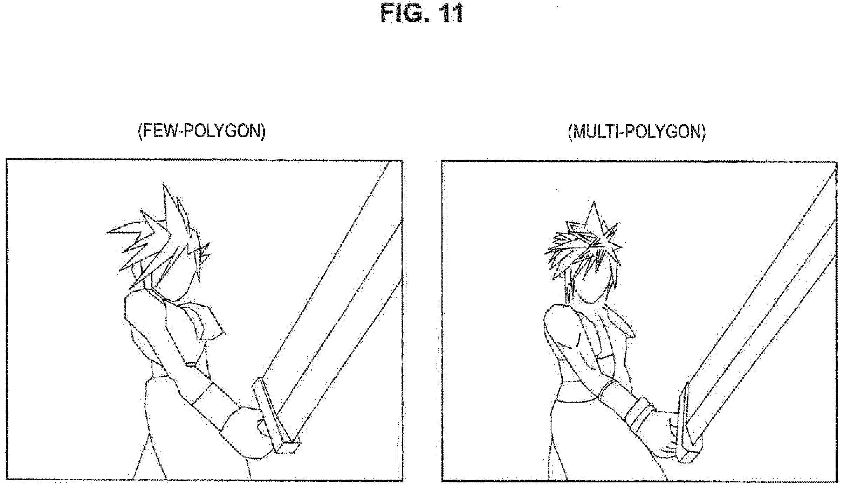

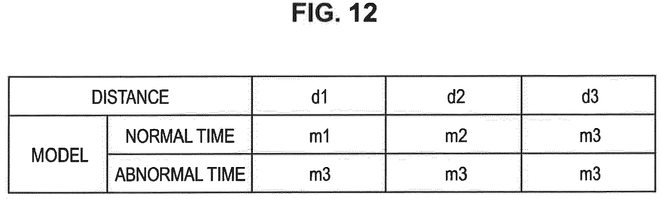

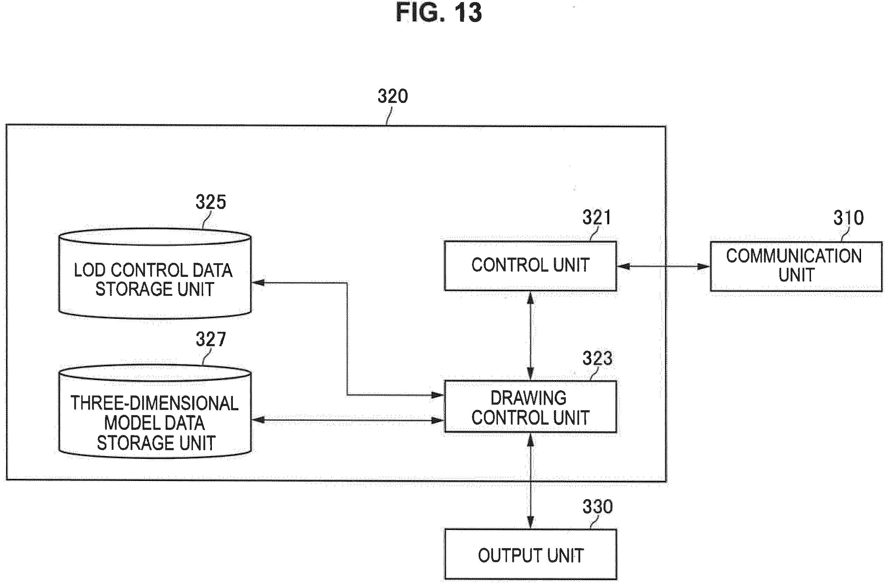

Hereinafter, as an example of such processing, a case of providing feedback for the user by changing definition of a display of virtual objects in the virtual world will be described on the basis of FIG. 11 to FIG. 13. Note that FIG. 11 is an explanatory diagram showing a display change in accordance with a difference in the number of polygons. FIG. 12 is a correspondence table between the distance from a camera and a model in LOD control of polygon model. FIG. 13 is a functional block diagram of a functional unit that performs LOD control of polygon model.

Usually, a game draws high-definition three-dimensional graphics using high-performance GPU and CPU in order for a user to be immersed in a virtual world. High definition provides the user with a feeling closer to the real world, whilst it is possible to cause the user to perceive that it is a virtual world by intentionally reducing definition to the contrary. As shown in FIG. 11, for example, since a model having a small number of polygons on the left deviates from objects existing in the real world as compared with a model having a large number of polygons on the right, a feeling of a virtual world can be emphasized.

As means for making a more realistic and graphic expression in three-dimensional graphics in this manner, a higher-definition polygon model and texture are used for drawing. However, if all of polygon models and texture in a game world are increased in definition at a time, burdens on GPU and a memory band increase. Therefore, usually in a game, a model having a large number of polygons (multi-polygon model) and a model having a small number of polygons (few-polygon model) are prepared in multiple stages for the same model, and a polygon model used for drawing is switched using a value called Level Of Detail (LOD) to reduce burdens. Usually, the distance from a camera and a LOD value are linked such that a model close to a position of a drawing camera draws a multi-polygon model and a distant model draws a few-polygon model. A similar technique is adopted for texture.

Here, in order to intentionally reduce definition, it is only necessary to change a threshold value for linking the distance and the LOD value. A correspondence table between the distance from a camera and a model in LOD control of polygon model is illustrated in FIG. 12. The distances from the camera are denoted as d1, d2, and d3, which shall have larger values in this order (d1<d2<d3). Indices of polygon models are denoted as m1, m2, and m3, which shall be models having a different number of polygons from one another. Here, the number of polygons shall be decreased in the order of m1, m2, and m3 (m1>m2>m3).

As shown in FIG. 12, at a usual time, the m1 model having the largest number of polygons is used when at the closer distance d1, and the m3 model having the smallest number of polygons is used when at the farther distance d3 or more. On the other hand, when issuing a warning to provide feedback for the user, the m3 model having the smallest number of polygons when placed at any distance shall be used. Accordingly, a model having a small number of polygons, i.e., having low-definition is presented as a virtual object provided in the virtual world.

Display change processing is performed in the feedback processing unit 320 of the feedback device 300 shown in FIG. 2, for example. Details thereof are shown in FIG. 13. The functional unit that performs the display change processing includes a control unit 321, a drawing control unit 323, a LOD control data storage unit 325, and a three-dimensional model data storage unit 327, as shown in FIG. 13.

The control unit 321 performs processing of feedback for the user on the basis of information concerning feedback received from the server 200 via the communication unit 310. In a case of providing feedback by a display change, the control unit 321 issues an instruction for a display change to the drawing control unit 323.

The drawing control unit 323 changes a display of a virtual object on the basis of the instruction for a display change from the control unit 321. The drawing control unit 323 refers to the LOD correspondence table shown in FIG. 12 stored in the LOD control data storage unit 325 to select three-dimensional model data for use in drawing on the basis of setting at the time of warning. The three-dimensional model data is stored in the three-dimensional model data storage unit 327. The three-dimensional model data storage unit 327 stores data necessary for three-dimensional drawing, such as polygon models and texture, as three-dimensional model data. The virtual object changed by the drawing control unit 323 is output to a video display appliance that the user is using, such as a head-mounted display, via the output unit 330.

It is possible to further reduce definition by similarly applying the above processing to texture as well. That is, by replacing a high-polygon model with high-resolution texture and a low-polygon model with low-resolution texture, it is possible to change LOD control of texture by processing similar to the foregoing.

### <5. Hardware configuration example>

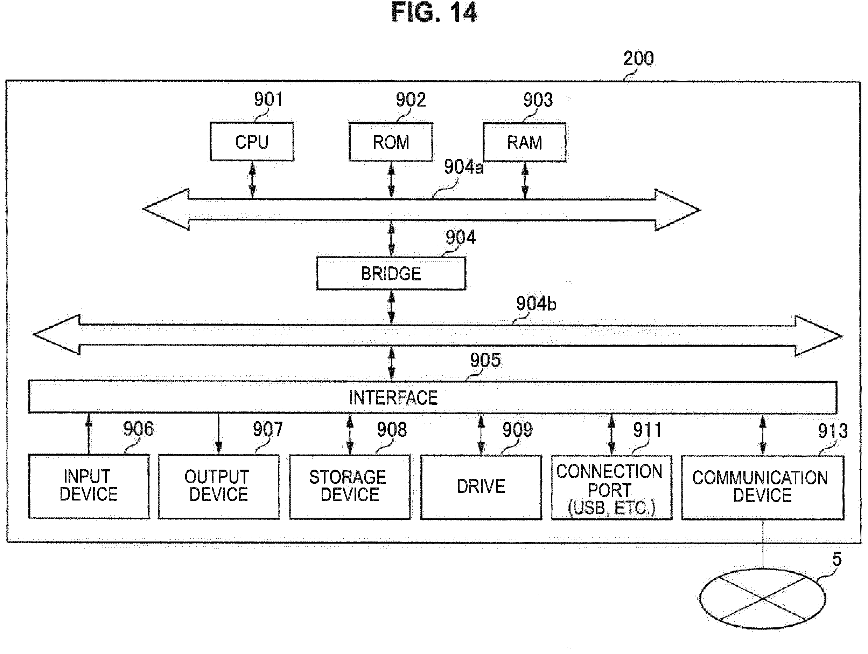

Finally, a hardware configuration example of the state notifying device 100, the server 200, and the feedback device 300 according to the above-described embodiment will be described. Since these devices can be configured similarly, the server 200 will be described below as an example. FIG. 14 is a hardware configuration diagram showing a hardware configuration of the server 200 according to the above embodiment.

The server 200 according to the present embodiment can be implemented as a processing device including a computer, as described above. As illustrated in FIG. 14, the server 200 is configured to include a central processing unit (CPU) 901, a read only memory (ROM) 902, a random access memory (RAM) 903, and a host bus 904a. In addition, the server 200 is configured to include a bridge 904, an external bus 904b, an interface 905, an input device 906, an output device 907, a storage device 908, a drive 909, a connection port 911, and a communication device 913.

The CPU 901 functions as an arithmetic processing device and a control device and controls the overall operation in the server 200 according to various programs. Further, the CPU 901 may be a microprocessor. The ROM 902 stores programs, operation parameters and the like used by the CPU 901. The RAM 903 temporarily stores programs used in execution of the CPU 901, parameters appropriately changed in the execution, and the like. These components are interconnected via the host bus 904a formed by a CPU bus or the like.

The host bus 904a is connected to the external bus 904b such as peripheral component interconnect/interface (PCI) bus through the bridge 904. Moreover, the host bus 904a, the bridge 904, and the external bus 904b are not necessarily configured as separate components, and the functions of them may be incorporated into a single bus.

The input device 906 is configured to include input means through which the user can input information, such as a mouse, a keyboard, a touch panel, a button, a microphone, a switch, and a lever, an input control circuit that generates an input signal on the basis of the input by the user and outputs it to the CPU 901, and the like. The output device 907 includes, in one example, a display device such as a liquid crystal display (LCD) device, an organic light emitting diode (OLED) device, or a lamp, and a speech output device such as a speaker.

The storage device 908 is an example of the storage unit of the server 200 and is a device for storing data. The storage device 908 may include a recording medium, a recording device that records data in the recording medium, a readout device that reads out data from the recording medium, a deletion device that deletes data recorded in the recording medium and the like. The storage device 908 drives a hard disk, and stores a program executed by the CPU 901 and various kinds of data.

The drive 909 is a reader-writer for a recording medium, and is built in the server 200 or is externally attached thereto. The drive 909 reads out information recorded in a mounted magnetic disk, optical disk, magneto-optical disc, or removable recording medium such as a semiconductor memory, and outputs the information to the RAM 903.

The connection port 911 is an interface connected to an external device and is a port for connecting an external device that is capable of transmitting data through, in one example, a universal serial bus (USB). Furthermore, the communication device 913 is, in one example, a communication interface formed by a communication device or the like for connecting to a communication network 5. Furthermore, the communication device 913 may be a communication device compatible with a wireless local area network (LAN), a communication device compatible with a wireless USB, or a wired communication device that communicates with wire.

The preferred embodiment(s) of the present disclosure has/have been described above with reference to the accompanying drawings, whilst the present disclosure is not limited to the above examples. A person skilled in the art may find various alterations and modifications within the scope of the appended claim.

### Reference Signs List

- 1: information processing system
- 100: state notifying device
- 110: each type of sensor
- 120: transmission processing unit
- 130: input unit
- 140: communication unit
- 200: server
- 210: communication unit
- 220: state determination unit
- 230: feedback decision unit
- 240: setting storage unit
- 300: feedback device
- 310: communication unit
- 320: feedback processing unit
- 321: control unit
- 323: drawing control unit
- 325: control data storage unit
- 327: dimensional model data storage unit

## Claims

1. An information processing system comprising:
a state notifying device (100);
a server (200);
a feedback device (300); and
a head mounted display configured to provide an augmented reality to a user to allow the user to experience a virtual world in which information of at least partially including a virtual object is provided,
wherein the state notifying device (100) comprises a plurality of sensors (110) for acquiring biological information as monitoring information, the biological information including heart rate and/or a blood pressure of the user,
wherein the state notifying device (100) is configured to transmit the monitoring information to the server (200),
wherein the server (200) comprises:
a state determination unit (220) configured to determine a user state of the user for determining whether feedback for the user is necessary or not on the basis of information received from the state notifying device (100), wherein the state determination unit (220) determines the user state on a basis of the monitoring information and on a basis of whether a value of the monitoring information acquired from the user exceeds a predetermined threshold value or not;
a feedback decision unit (230) configured to, when it is determined by the state determination unit (220) that feedback is necessary,
decide feedback for the user on a basis of the user state of the user, wherein the feedback decision unit (230) decides feedback for the user among an additional information type feedback, an information reduced type feedback, and a forced termination type feedback, wherein stronger feedback is provided for the user in the order of the additional information type feedback, the information reduced type feedback, and the forced termination type feedback, wherein the additional information type feedback is additional information for the user to differentiate between the virtual world and the real world and includes adding frames to virtual objects in the virtual world or reducing a number of polygons of the virtual objects, wherein the information reduced type feedback includes deleting a virtual object display in the virtual world and switching to the screen of the real world alone, and wherein the forced termination type feedback includes forcibly shutting down a content of the virtual world, wherein urgency of the feedback is determined on the basis of the heart rate or the blood pressure of the user measured by each sensor (110) of the state notifying device (100), wherein the higher the urgency the stronger is the feedback, and
transmit decided feedback information to the feedback device (300) configured to be capable of notifying the user,
wherein the feedback device (300) is configured to provide feedback for the user on the basis of the feedback information received from the server (200),
wherein the feedback device (300) comprises:
a feedback processing unit (320) that is configured to perform display change processing for changing a display of the virtual world in accordance with the feedback information; and
an output unit (330) that is configured to output information to the head mounted display on the basis of a result of the processing performed by the feedback processing unit (320).

## Patentansprüche

1. Informationsverarbeitungssystem, umfassend:
eine Zustandsbenachrichtigungsvorrichtung (100);
einen Server (200);
eine Feedback-Vorrichtung (300); und
ein Head-Mounted-Display, das ausgestaltet ist, um einem Benutzer eine Augmented Reality bereitzustellen, um dem Benutzer das Erleben einer virtuellen Welt zu ermöglichen, worin Informationen bereitgestellt werden, die mindestens teilweise ein virtuelles Objekt einschließen,
wobei die Zustandsbenachrichtigungsvorrichtung (100) eine Vielzahl von Sensoren (110) umfasst, um biologische Informationen als Überwachungsinformationen zu erfassen, wobei die biologischen Informationen eine Herzfrequenz und/oder einen Blutdruck des Benutzers einschließen,
wobei die Zustandsbenachrichtigungsvorrichtung (100) ausgestaltet ist, um die Überwachungsinformation an den Server (200) zu übermitteln,
wobei der Server (200) umfasst:
eine Zustandsbestimmungseinheit (220), die ausgestaltet ist, um einen Benutzerzustand des Benutzers zu bestimmen, um basierend auf Informationen, die von der Zustandsbenachrichtigungsvorrichtung (100) empfangen wurden, zu bestimmen, ob Feedback an den Benutzer erforderlich ist oder nicht, wobei die Zustandsbestimmungseinheit (220) den Benutzerzustand basierend auf den Überwachungsinformationen und basierend darauf bestimmt, ob ein Wert der von dem Benutzer erfassten Überwachungsinformationen einen vorbestimmten Schwellenwert überschreitet oder nicht;
eine Feedback-Entscheidungseinheit (230), die ausgestaltet ist, um folgendes auszuführen, wenn durch die Zustandsbestimmungseinheit (220) bestimmt wird, dass Feedback erforderlich ist:
Entscheiden von Feedback an den Benutzer basierend auf dem Benutzerzustand des Benutzers, wobei die Feedback-Entscheidungseinheit (230) Feedback an den Benutzer innerhalb von einem Feedback vom zusätzlichen Informationstyp, Feedback vom informationsreduzierten Typ und Feedback vom Typ forcierte Beendigung umfasst, wobei dem Benutzer in der Reihenfolge von Feedback vom zusätzlichen Informationstyp, Feedback vom informationsreduzierten Typ und Feedback vom Typ forcierte Beendigung stärkeres Feedback bereitgestellt wird, wobei das Feedback vom zusätzlichen Informationstyp zusätzliche Informationen für den Benutzer sind, um zwischen der virtuellen Welt und der realen Welt zu differenzieren, und Zufügen von Frames zu virtuellen Objekten in der virtuellen Welt oder Reduzieren einer Anzahl von Vielecken der virtuellen Objekte einschließt, wobei Feedback vom informationsreduzierten Typ Löschen einer virtuellen Objektanzeige in der virtuellen Welt und Umschalten zu dem Bildschirm der realen Welt allein einschließt, und wobei Feedback vom forcierten Beendigungstyp forciertes Herunterfahren eines Inhalts der virtuellen Welt einschließt, wobei die Dringlichkeit des Feedbacks basierend auf der Herzfrequenz oder dem Blutdruck des Benutzers bestimmt wird, gemessen durch jeden Sensor (110) der Zustandsbenachrichtigungsvorrichtung (100), wobei das Feedback umso stärker ist, je höher die Dringlichkeit ist, und
Übermitteln von entschiedenen Feedback-Informationen an die Feedback-Vorrichtung (300), die so ausgestaltet ist, dass sie imstande ist, den Benutzer zu benachrichtigen,
wobei die Feedback-Vorrichtung (300) ausgestaltet ist, um basierend auf von dem Server (200) empfangenen Feedback-Informationen Feedback an den Benutzer bereitzustellen.
wobei die Feedback-Vorrichtung (300) umfasst:
eine Feedback-Verarbeitungseinheit (320), die ausgestaltet ist, um Anzeigeveränderungsverarbeitung zum Verändern einer Anzeige der virtuellen Welt gemäß den Feedback-Informationen bereitzustellen; und
eine Ausgabeeinheit (330), die ausgestaltet ist, um basierend auf einem Ergebnis der Verarbeitung, die durch die Feedback-Verarbeitungseinheit (320) durchgeführt wurde, Informationen an das Head-Mounted-Display auszugeben.

## Revendications

1. Système de traitement d'informations comprenant :
un dispositif de notification d'état (100) ;
un serveur (200) ;
un dispositif de rétroaction (300) ; et
un visiocasque configuré pour fournir une réalité augmentée à un utilisateur pour permettre à l'utilisateur de faire l'expérience d'un monde virtuel dans lequel des informations comprenant au moins partiellement un objet virtuel sont fournies,
dans lequel le dispositif de notification d'état (100) comprend une pluralité de capteurs (110) pour acquérir des informations biologiques en tant qu'informations de surveillance, les informations biologiques comprenant la fréquence cardiaque et/ou une pression artérielle de l'utilisateur,
dans lequel le dispositif de notification d'état (100) est configuré pour transmettre les informations de surveillance au serveur (200),
dans lequel le serveur (200) comprend :
une unité de détermination d'état (220) configurée pour déterminer un état d'utilisateur de l'utilisateur pour déterminer si une rétroaction pour l'utilisateur est nécessaire ou non sur la base d'informations reçues du dispositif de notification d'état (100), dans lequel l'unité de détermination d'état (220) détermine l'état d'utilisateur sur la base des informations de surveillance et sur la base du fait qu'une valeur des informations de surveillance acquises auprès de l'utilisateur dépasse ou non une valeur de seuil prédéterminée ;
une unité de décision de rétroaction (230) configurée pour, lorsqu'il est déterminé par l'unité de détermination d'état (220) qu'une rétroaction est nécessaire,
décider d'une rétroaction pour l'utilisateur sur la base de l'état d'utilisateur de l'utilisateur, dans lequel l'unité de décision de rétroaction (230) décide d'une rétroaction pour l'utilisateur parmi une rétroaction de type informations supplémentaires, une rétroaction de type informations réduites et une rétroaction de type arrêt forcé, dans lequel une rétroaction plus forte est fournie à l'utilisateur dans l'ordre suivant : la rétroaction de type informations supplémentaires, la rétroaction de type informations réduites et la rétroaction de type arrêt forcé, dans lequel la rétroaction de type informations supplémentaires représente des informations supplémentaires permettant à l'utilisateur de différencier le monde virtuel du monde réel et comprend l'ajout de trames à des objets virtuels dans le monde virtuel ou la réduction d'un nombre de polygones des objets virtuels, dans lequel la rétroaction de type informations réduites comprend la suppression de l'affichage d'un objet virtuel dans le monde virtuel et le passage à l'écran du monde réel seul, et dans lequel la rétroaction de type arrêt forcé comprend l'arrêt forcé d'un contenu du monde virtuel, dans lequel l'urgence de la rétroaction est déterminée sur la base de la fréquence cardiaque ou de la pression artérielle de l'utilisateur mesurée par chaque capteur (110) du dispositif de notification d'état (100), dans lequel plus l'urgence est élevée, plus la rétroaction est forte, et
transmettre les informations de rétroaction décidées au dispositif de rétroaction (300) configuré pour être capable de notifier l'utilisateur,
dans lequel le dispositif de rétroaction (300) est configuré pour fournir une rétroaction à l'utilisateur sur la base des informations de rétroaction reçues du serveur (200),
dans lequel le dispositif de rétroaction (300) comprend :
une unité de traitement de rétroaction (320) qui est configurée pour effectuer un traitement de changement d'affichage pour changer un affichage du monde virtuel en fonction des informations de rétroaction ; et
une unité de sortie (330) qui est configurée pour sortir des informations vers le visiocasque sur la base d'un résultat du traitement effectué par l'unité de traitement de rétroaction (320).
